(19) Europäisches Patentamt
European Patent Office
Office européen des brevets

(11) Veröffentlichungsnummer: **0 198 791**
**B1**

# (12) EUROPÄISCHE PATENTSCHRIFT

(45) Veröffentlichungstag der Patentschrift: **25.01.89**

(51) Int. Cl.⁴: **C07C 43/29**, C07C 41/30, A01N 31/14

(21) Anmeldenummer: **86730064.2**

(22) Anmeldetag: **08.04.86**

(54) **Difluorcyclopropanderivate, Verfahren zu ihrer Herstellung sowie Schädlingsbekämpfungsmittel enthaltend diese Verbindungen.**

(30) Priorität: **09.04.85 DE 3513041**

(43) Veröffentlichungstag der Anmeldung: **22.10.86 Patentblatt 86/43**

(45) Bekanntmachung des Hinweises auf die Patenterteilung: **25.01.89 Patentblatt 89/4**

(84) Benannte Vertragsstaaten: **AT BE CH DE FR GB IT LI LU NL SE**

(56) Entgegenhaltungen:
**EP-A- 0 094 085**
**EP-A- 0 104 908**
**EP-A- 0 125 204**
**EP-A- 0 136 451**
**WO-A-84/04298**
**GB-A- 2 120 664**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT Berlin und Bergkamen, Müllerstrasse 170/178 Postfach 65 03 11, D-1000 Berlin 65(DE)**

(72) Erfinder: **Franke, Heinrich, Dr., Fabeckstrasse 38, D-1000 Berlin 37(DE)**
Erfinder: **Jopplen, Hartmut, Dr., Juttastrasse 18, D-1000 Berlin 37(DE)**
Erfinder: **Franke, Helga, Dr., Karl-Stieler-Strasse 2b, D-1000 Berlin 41(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die Erfindung betrifft neue Difluorcyclopropanderivate, ihre Herstellung sowie Schädlingsbekämpfungsmittel auf Basis dieser Wirkstoffe.

Cyclopropanderivate mit insektizider Wirkung sind bereits beschrieben worden, wie z.B. in EP 94 085 (1), EP 104 908 (2) und DE-OS 3 317 908 (3).

In (1) werden insektizide und akarizide Verbindungen der allgemeinen Formel

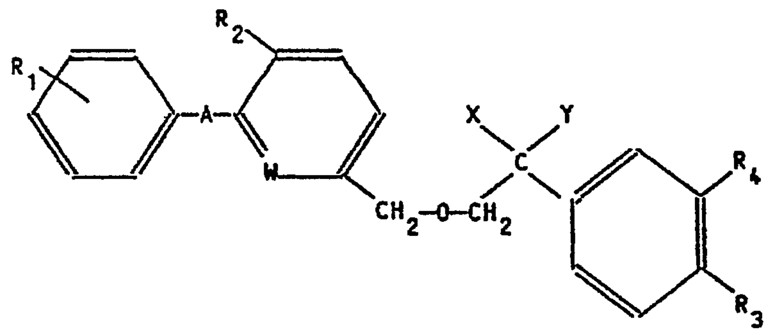

beschrieben, worin unter einer Vielzahl von genannten Verbindungen auch vier Verbindungen enthalten sind, in denen $R_1$ und $R_4$ Wasserstoff; A Sauerstoff; $R_2$ Wasserstoff oder Fluor; W eine $CH_2$-Gruppe; X und Y zusammen eine 1,1-Difluorethyl-Gruppe und $R_3$ eine Ethoxy-Gruppe oder ein Cl-Atom bedeuten.

In (2) werden arthropodizide Verbindungen der allgemeinen Formel

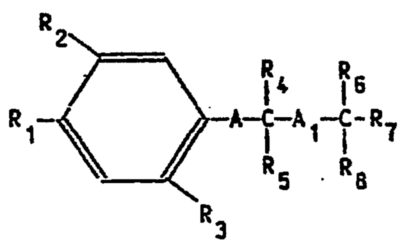

beschrieben, worin neben einer Vielzahl von Verbindungen auch die Verbindungen der in (1) genannten Insektizide und Akarizide enthalten sind und von denen $R_1$ für ein Cl-Atom oder eine Ethyloxy-Gruppe, $R_2$ und $R_3$ Wasserstoff, A für Tetrafluorcyclobutan, Difluorcyclopropan oder Dichlorcyclopropan, $R_4$ und $R_5$ für Wasserstoff, $A_1$ für ein O-Atom oder eine $CH_2$-Gruppe steht oder, falls $A_1$ eine $CH_2$-Gruppe darstellt, bedeutet $R_4$ und $R_5$ gemeinsam Sauerstoff, $R_6$ und $R_8$ steht für Wasserstoff und $R_7$ für eine 3-Phonxyphenyl- oder 4-Fluorphenoxzphenylgruppe.

In (3) werden als Insektizide Verbindungen der Formel

$Ar{-}CR^1R^2{-}CH_2{-}CH_2R^3$

angegeben, wobei Ar einen gegebenenfalls substituierten Phenyl- oder Naphthylring, $R^1$ Methyl, Ethyl oder Isopropyl, $R^2$ Wasserstoff oder Methyl oder $R^1$ und $R^2$ zusammen mit dem C-Atom, an welches sie gebunden sind, gegebenenfalls substituiertes Cycloalkyl und $R^3$ den Rest eines Alkohols der Formel $R^3OH$ bedeuten.

Unter den beschriebenen Verbindungen, bei denen $R^1$ und $R^2$ eine Cycloalkylgruppe bilden, befindet sich eine, die geminal mit zwei Chloratomen substituiert ist.

Es wurde nun gefunden, daß eine Reihe von Verbindungen, in denen dieses Chlor durch Fluor ersetzt wird und somit Difluorcyclopropanderivate der allgemeinen Formel I darstellen

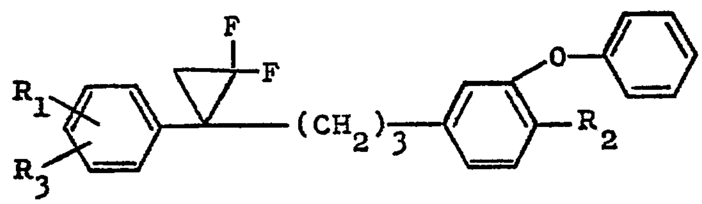 (I),

in der $R_1$ und $R_3$ gleich oder verschieden sind und Wasserstoff, Halogen, $C_{1-4}$-Alkoxy, Fluor-substitu-

iertes $C_{1-4}$-Alkoxy, $C_{1-4}$-Alkyl oder Trifluormethyl und $R_2$ Wasserstoff oder Fluor bedeuten, sich durch besonders wertvolle insektizide und akarizide Eigenschaften auszeichnen.

Unter Halogen ist insbesondere Fluor, Chlor und Brom zu verstehen. Unter Alkyl bzw. Alkoxy sind sowohl geradwie verzweigtkettige aliphatische Reste zu verstehen, wie z.B. Methyl, Ethyl, Propyl, i-Propyl oder tert.-Butyl.

Bevorzugt sind solche Verbindungen der Formel I, bei denen $R_1$ sich in 4-Stellung befindet und Chlor oder Ethoxy bedeutet und $R_3$ Wasserstoff ist.

Beispielsweise sind dies die Verbindungen
1-(4-Chlorphenyl)-2,2-difluor-1-[3-(3-phenoxyphenyl)-propyl]-cyclopropan,
2,2-Difluor-1-(4-ethoxyphenyl)-1-[3-(3-phenoxyphenyl)-propyl]-cyclopropan,
1-(4-Chlorphenyl)-2,2-difluor-1-[3-(4-fluor-3-phenoxyphenyl)-propyl]-cyclopropan und
2,2-Difluor-1-(4-ethoxyphenyl)-1-[3-(4-fluor-3-phenoxyphenyl)-propyl]-cyclopropan.

Die Anwendung der erfindungsgemäßen Verbindungen als Schädlingsbekämpfungsmittel kann in Konzentrationen von 0,0005 bis 5,0%, vorzugsweise von 0,001 bis 0,1%, erfolgen, worunter das Gewicht in Gramm Wirkstoff in 100 ml Zubereitung zu verstehen ist.

Die erfindungsgemäßen Verbindungen können entweder allein, in Mischung miteinander oder mit anderen insektiziden Wirkstoffen angewendet werden. Gegebenenfalls können andere Pflanzenschutz- oder Schädlingsbekämpfungsmittel, wie zum Beispiel Insektizide, Akarizide oder Fungizide, je nach dem gewünschten Zweck zugesetzt werden.

Eine Förderung der Wirkungsintensität und der Wirkungsgeschwindigkeit kann zum Beispiel durch wirkungssteigernde Zusätze, wie organische Lösungsmittel, Netzmittel und Öle, erzielt werden. Solche Zusätze lassen daher gegebenenfalls eine Verringerung der Wirkstoffdosierung zu.

Als Mischungspartner können außerdem Phospholipide verwendet werden, z.B. solche aus der Gruppe Phosphatidylcholin, hydriertes Phosphatidylcholin, Phosphatidylethanolamin, N-Acyl-phosphatidylethanolamin, Phosphatidylinosit, Phosphatidylserin, Lysolecithin und Phosphatidylglycerol.

Zweckmäßig werden die Wirkstoffe oder deren Mischungen in Form von Zubereitungen, wie Pulvern, Streumitteln, Granulaten, Lösungen, Emulsionen oder Suspensionen, unter Zusatz von flüssigen und/oder festen Trägerstoffen bzw. Verdünnungsmitteln und gegebenenfalls als Haft-, Netz-, Emulgier- und/oder Dispergierhilfsmitteln angewandt.

Geeignete flüssige Trägerstoffe sind zum Beispiel aliphatische und aromatische Kohlenwasserstoffe, wie Benzol, Toluol, Xylol, Cyclohexanon, Isophoron, Acetophenon, Dimethylsulfoxid, Dimethylformamid, weiterhin Mineralölfraktionen und Pflanzenöle.

Als feste Trägerstoffe eignen sich Mineralien, zum Beispiel Tonsil, Silicagel, Talkum, Kaolin, Attapulgit, Kalkstein, Kieselsäure und pflanzliche Produkte, wie z.B. Mehle.

An oberflächenaktiven Stoffen sind beispielsweise zu nennen Calciumligninsulfonat, Polyethylenalkylphenylether, Naphthalinsulfonsäuren und deren Salze, Phenolsulfonsäuren und deren Salze, Formaldehydkondensate, Fettalkoholsulfate sowie substituierte Benzolsulfonsäuren und deren Salze.

Der Anteil an Wirkstoffen in den verschiedenen Zubereitungen kann in weiten Grenzen variieren. Beispielsweise enthalten die Mittel etwa 10 bis 90 Gewichtsprozent Wirkstoff, etwa 90 bis 10 Gewichtsprozent flüssige oder feste Trägerstoffe sowie gegebenenfalls bis zu 20 Gewichtsprozent oberflächenaktive Stoffe.

Die Ausbringung der Mittel kann in üblicher Weise erfolgen, mit Wasser als Träger in Spritzbrühmengen von etwa 100 bis 3000 Liter/ha. Eine Anwendung der Mittel im sogenannten Low-Volume- und Ultra-Low-Volume-Verfahren ist ebenso möglich wie ihre Applikation in Form von sogenannten Mikrogranulaten.

Die Herstellung dieser Zubereitungen kann in an sich bekannter Art und Weise, z.B. durch Mahl- oder Mischverfahren, durchgeführt werden. Gewünschtenfalls können die Einzelkomponenten auch erst kurz vor ihrer Verwendung gemischt werden, wie es z.B. im sogenannten Tankmixverfahren in der Praxis gemacht wird.

Zur Herstellung der Zubereitungen werden die folgenden Bestandteile eingesetzt:

a) 80 Gewichtsprozent Wirkstoff
15 Gewichtsprozent Kaolin
5 Gewichtsprozent oberflächenaktive Stoffe auf Basis des Natriumsalzes des N-Methyl-N-oleyltaurins und des Calciumsalzes der Ligninsulfonsäure
b) 45 Gewichtsprozent Wirkstoff
5 Gewichtsprozent Natriumaluminiumsilikat
15 Gewichtsprozent Cetylpolyglykolether mit 8 Mol Ethylenoxid
2 Gewichtsprozent Spindelöl
10 Gewichtsprozent Polyethylenglykol
23 Teile Wasser
c) 20 Gewichtsprozent Wirkstoff
35 Gewichtsprozent Tonsil
8 Gewichtsprozent Ca-Salz der Ligninsulfonsäure
2 Gewichtsprozent Natriumsalz des N-Methyl-N-oleyltaurins

35 Gewichtsprozent Kieselsäure
d) 20 Gewichtsprozent Wirkstoff
75 Gewichtsprozent Isophoron
5 Gewichtsprozent Kombinationsemulgator aus Calciumphenylsulfonat und Fettalkoholpolyglykolether

Die erfindungsgemäßen Verbindungen lassen sich herstellen, indem man eine Verbindung der allgemeinen Formel II

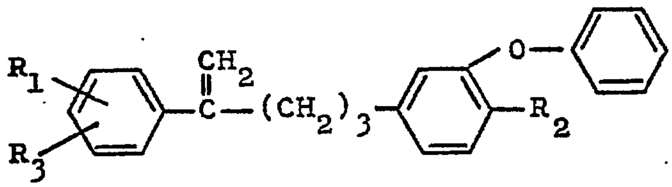 (II)

mit Difluorcarben umsetzt, wobei $R_1$, $R_2$ und $R_3$ die oben genannte Bedeutung haben.

Das Verfahren wird zweckmäßigerweise so durchgeführt, daß das Difluorcarben in der Reaktionsmischung erzeugt wird. Zur Erzeugung von Difluorcarben kommen im Prinzip alle Methoden in Frage. Eine bevorzugte Ausführungsform ist die Zersetzung von Natrium-chlordifluoracetat, in Diglyme gelöst, bei ca. 165 C in Gegenwart des Ausgangsmaterials.

Geeignet sind auch andere polare Lösungsmittel, die nicht mit Difluorcarben reagieren, wie Dimethylformamid oder Sulfolan.

Das Ausgangsmaterial der Formel II läßt sich in einer dreistufigen Synthese herstellen. In der ersten Stufe wird ein Nitril der Formel

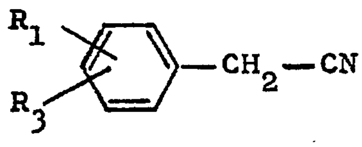

mit einem Alkylierungsmittel der Formel

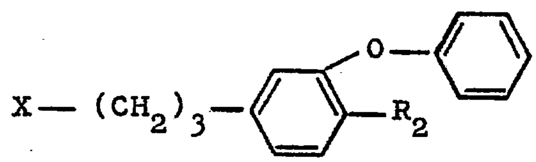

in Gegenwart einer Base umgesetzt.

In der zweiten Stufe wird dann das so erhaltene Alkylierungsprodukt in Gegenwart von Alkalihydroxid mit Luftsauerstoff unter Verlust der Cyanogruppe zum Keton oxydiert und anschließend in der dritten Stufe das Keton mit Triphenylmethylen-phosphoran in einer Wittig-Reaktion zum Olefin umgesetzt.

$R_1$, $R_2$ und $R_3$ haben die in Formel I angegebene Bedeutung, und X bedeutet eine Fluchtgruppe wie Cl, Br, Mesylat oder Tosylat.

Das folgende Beispiel erläutert die Herstellung der erfindungsgemäßen Verbindungen.

<u>Beispiel 1</u>

1-(4-Chlorphenyl)-2,2-difluor-1-[3-(4-fluor-3-phenoxyphenyl)-propyl]-cyclopropan

3,0 g (20 mMol) Natriumchlordifluoracetat, gelöst in 15 ml Diglyme, werden innerhalb von 60 Minuten zu einer siedenden Lösung (Badtemperatur 200°C) von 2,76 g (7,5 mMol) 2-(4-Chlorphenyl)-5-(4-fluor-3-phenoxyphenyl)-penten-(1) in 15 ml Diglyme getropft. Anschließend wird noch 30 Minuten weitergekocht. Nach dem Abkühlen wird auf Wasser gegossen, dreimal mit Ether extrahiert, mit Wasser gewaschen und mit Magnesiumsulfat getrocknet. Den Eindampfrückstand chromatographiert man an 100 g Kieselgel mit einem Gemisch von Ether/Hexan 1:9. Nach dem Eindampfen verbleiben 2,8 g Produkt (90% der Theorie).
$n_D20$ : 1,5598

Das Ausgangsmaterial wird auf folgende Weise hergestellt:

1. 2-(4-Chlorphenyl)-5-(4-fluor-3-phenoxyphenyl)-valeronitril
Zu einer Mischung von 30,6 g (0,2 Mol) 4-Chlorbenzylcyanid, 50 ml 50%iger Natronlauge und 0,5 g Tetrabutylammoniumbromid werden unter starkem Rühren 23,2 g (0,075 Mol) 1-Brom-3-(4-fluor-3-phenoxyphenyl)-propan zugetropft; durch leichtes Kühlen hält man die Temperatur bei 30°C. Nach dreistündigem Rühren wird auf Eiswasser gegossen, zweimal mit Ether extrahiert, mit Wasser gewaschen, mit Magnesiumsulfat getrocknet und eingedampft. Überschüssiges Benzylcyanid wird am Kugelrohr bei 150 C und 0,1 Torr abdestilliert. Anschließend wird an Kieselgel chromatographiert.
Ausbeute: 27,0 g = 95% der Theorie $n_D20$ : 1,5752
2. 1-(4-Chlorphenyl)-4-(4-fluor-3-phenoxyphenyl)-butan-1-on
27,0 g (0,071 Mol) des vorstehenden Nitrils, 220 ml Toluol, 220 ml 50%ige Natronlauge und 1,5 g Benzyltriethylammoniumchlorid (TEBA) werden bei Raumtemperatur unter Überleiten von Luft stark gerührt. Nach 2,5 Stunden ist die Reaktion beendet. Man gießt auf Eiswasser, extrahiert dreimal mit Ether, wäscht mit Wasser und trocknet. Der Eindampfrückstand ist nach DC einheitlich und wird ohne Reinigung weiter umgesetzt.
Ausbeute: 18,9 g = 72% der Theorie.
3. 2-(4-Chlorphenyl)-5-(4-fluor-3-phenoxyphenyl)-penten-(1)
4,2 g (0,095 Mol) 55%ige Natriumhydrid-Dispersion werden durch mehrmaliges Waschen mit Toluol vom Öl befreit und dann mit 150 ml DMSO versetzt. Man erwärmt auf 80°C, bis sich alles gelöst hat (ca. 30 Minuten). Bei Raumtemperatur werden 36,5 g (0,09 Mol) Methyltriphenylphosphoniumjodid zugegeben und gerührt, bis vollständige Lösung eingetreten ist. Dann werden 18,8 g (0,051 Mol) des Ketons, gelöst in 50 ml DMSO, zugetropft und 12 Stunden bei Raumtemperatur gerührt. Man gießt auf Eiswasser, extrahiert dreimal mit Ether, wäscht die organischen Phasen mit Wasser und trocknet mit Magnesiumsulfat. Der Eindampfrückstand wird an Kieselgel chromatographiert.
Ausbeute: 12,6 g = 68% der Theorie $n_D20$ : 1,5898

In analoger Weise werden die folgenden Verbindungen hergestellt:

| Beispiel Nr. | Verbindung | Physikalische Konstante $n_D20$ |
|---|---|---|
| 2 | 1-(4-Chlorphenyl)-2,2-difluor-1-[3-(3-phenoxyphenyl)-propyl]-cyclopropan | 1,5672 |
| 3 | 2,2-Difluor-1-(4-ethoxyphenyl)-1-[3-(3-phenoxyphenyl)-propyl]-cyclopropan | 1,5580 |
| 4 | 2,2-Difluor-1-(4-ethoxyphenyl)-1-[3-(4-fluor-3-phenoxyphenyl)-propyl]-cyclopropan | 1,5492 |
| 5 | 2,2-Difluor-1-(4-methoxyphenyl)-1-[3-(3-phenoxyphenyl)-propyl]-cyclopropan | 1,5623 |
| 6 | 2,2-Difluor-1-(4-difluormethoxyphenyl)-1-[3-(4-fluor-3-phenoxyphenyl)-propyl]-cyclopropan | 1,5325 |
| 7 | 2,2-Difluor-1-[3-(4-fluor-3-phenoxyphenyl)-propyl]-1-(4-methoxyphenyl)-cyclopropan | 1,5546 |
| 8 | 2,2-Difluor-1-[4-(2-fluorethoxy)-phenyl]-1-[3-(4-fluor-3-phenoxyphenyl)-propyl]-cyclopropan | 1,5470 |
| 9 | 2,2-Difluor-1-(4-fluorphenyl)-1-[3-(4-fluor-3-phenoxyphenyl)-propyl]-cyclopropan | 1,5433 |
| 10 | 1-(4-Bromphenyl)-2,2-difluor-1-[3-(4-fluor-3-phenoxyphenyl)-propyl]-cyclopropan | 1,5695 |
| 11 | 2,2-Difluor-1-[3-(4-fluor-3-phenoxyphenyl)-propyl]-1-(4-trifluormethylphenyl)-cyclopropan | 1,5220 |
| 12 | 2,2-Difluor-1-[3-(4-fluor-3-phenoxyphenyl)-propyl]-1-(4-prop-2-yloxyphenyl)-cyclopropan | 1,5466 |
| 13 | 2,2-Difluor-1-[3-(4-fluor-3-phenoxyphenyl)-propyl]-1-(4-propyloxyphenyl)-cyclopropan | 1,5449 |
| 14 | 1-(4-Butyloxyphenyl)-2,2-difluor-1-[3-(4-fluor-3-phenoxyphenyl)-propyl]-cyclopropan | 1,5412 |
| 15 | 2,2-Difluor-1-[3-(4-fluor-3-phenoxyphenyl)-propyl]-1-(4-methylphenyl)-cyclopropan | 1,5501 |
| 16 | 1-(3,4-Dichlorphenyl)-2,2-difluor-1-[3-(4-fluor-3-phenoxyphenyl)-propyl] | 1,5673 |
| 17 | 2,2-Difluor-1-(3-fluor-4-methoxyphenyl)-1-[3-(4-fluor-3-phenoxyphenyl)-propyl]-cyclopropan | 1,5468 |
| 18 | 2,2-Difluor-1-(4-ethoxy-3-fluorphenyl)-1-[3-(4-fluor-3-phenoxyphenyl)-propyl]-cyclopropan | 1,5432 |
| 19 | 2,2-Difluor-1-[4-(2-fluorethoxy)-3-fluorphenyl]-1-[3-(4-fluor-3-phenoxyphe nyl)-propyl]-cyclopropan | 1,5409 |
| 20 | 2,2-Difluor-1-phenyl-1-[3-(3-phenoxyphenyl)-propyl]-cyclopropan | 1,5643 |
| 21 | 2,2-Difluor-1-(4-ethylphenyl)-1-[3-(3-phenoxyphenyl)-propyl]-cyclopropan | 1,5589 |
| 22 | 2,2-Difluor-1-(4-n-propylphenyl)-1-[3-(3-phenoxyphenyl)-propyl]-cyclopropan | 1,5595 |
| 23 | 2,2-Difluor-1-(4-tert.-butylphenyl)-1-[3-(3-phenoxyphenyl)-propyl]-cyclopropan | 1,5427 |
| 24 | 2,2-Difluor-1-phenyl-1-[3-(4-fluor-3-phenoxyphenyl)-propyl]-cyclopropan | 1,5513 |
| 25 | 2,2-Difluor-1-(4-ethylphenyl)-1-[3-(4-fluor-3-phenoxyphenyl)-propyl]-cyclopropan | 1,5462 |
| 26 | 2,2-Difluor-1-[4-propylphenyl]-1-[3-(4-fluor-3-phenoxyphenyl)-propyl]-cyclopropan | 1,5482 |
| 27 | 2,2-Difluor-1-(4-tert.-butoxyphenyl)-1-[3-(4-fluor-3-phenoxyphenyl)-propyl]-cyclopropan | 1,5229 |

Anwendungsbeispiel 1

Abtötende Wirkung auf Larven (L3) des Mexikanischen Bohnenkäfers (Epilachna varivestis)

Die Verbindungen werden als wässrige Emulsionen mit der Wirkstoffkonzentration 0,0064% eingesetzt. In diese Wirkstoffzubereitungen werden Buschbohnenpflanzen (Phaseolus vulgaris) im Primärblattstadium getaucht. Pro Versuchsglied werden zwei Pflanzenstengel mit insgesamt 4 Primärblättern in mit Wasser gefüllte Glasvasen eingestellt und in Plexiglaszylindern eingekäfigt. Danach werden je 5 Larven des Mexikanischen Bohnenkäfers (Epilachna varivestis) im 3. Larvenstadium in die Glaszylinder eingezählt und für 3 Tage darin gehalten. Kriterium für die Wirkungsbeurteilung ist die Sterblichkeit der Larven nach 3 Tagen.

Es ergab sich, daß die erfindungsgemäßen Verbindungen gemäß den Beispielen 1–12, 14–18 und 20–22 eine 100%ige Wirkung (Mortalität) zeigten, währenddessen die Verbindung 2,2-Dichlor-1-(4-chlorphenyl)-1-[3-(3-phenoxyphenyl)-propyl]-cyclopropan (Nr. 50 in DE-OS 3 317 908) nur eine 90%ige Wirkung aufwies.

Anwendungsbeispiel 2

Abtötende Wirkung auf Junglarven der Kohlschabe (Plutella xylostella)

Die Verbindungen werden als wässrige Emulsionen mit der Wirkstoffkonzentration 0,0064% eingesetzt. Mit diesen Wirkstoffzubereitungen werden Blumenkohlblättchen in Polystyrol-Petrischalen (4 mg Spritzbrühmenge/cm$^2$) gespritzt. Nach dem Antrocknen der Spritzbeläge werden in jede Petrischale 10 Jungraupen der Kohlschabe (Plutella xylostella) eingezählt und für 2 Tage in den geschlossenen Petrischalen dem behandelten Futter exponiert. Kriterium für die Wirkungsbeurteilung ist die Sterblichkeit der Raupen in% nach 2 Tagen.

Es ergab sich, daß die erfindungsgemäßen Verbindungen gemäß Beispiel 1, 2, 4–7, 9–11, 15, 16, 18 und 19 eine 100%ige Wirkung (Mortalität) zeigten, währenddessen Nr. 50 (DE-OS 3 317 908) nur eine 20%ige Wirkung aufwies.

Anwendungsbeispiel 3

Abtötende Wirkung auf Larven (L2) des Ägyptischen Baumwollwurmes (Spodoptera littoralis)

Die erfindungsgemäßen Verbindungen werden als wässrige Emulsionen mit der Wirkstoffkonzentration 0,0064% eingesetzt. Mit diesen Wirkstoffzubereitungen werden je ein Fiederblattpaar der Puffbohne (Vicia faba) sowie 10 Larven (L2) des Ägyptischen Baumwollwurmes (Spodoptera littoralis) pro Versuchsglied mit 4 mg Spritzbrühe/cm$^2$ in Polystyrol-Petrischalen dosiert gespritzt. Die geschlossenen Petrischalen werden dann im Labor unter Langtagbedingungen für 2 Tage aufgestellt. Kriterium für die Wirkungsbeurteilung ist die Mortalität der Larven in% nach 2 Tagen.

Es ergab sich, daß die erfindungsgemäßen Verbindungen gemäß den Beispielen 1–5, 7–9, 11–13, 15 und 17–19 eine 100%ige Wirkung (Mortalität) zeigten, währenddessen die Verbindung Nr. 50 (DE-OS 3 317 908) keine Wirkung (0%) aufwies.

Anwendungsbeispiel 4

Abtötende Wirkung auf bewegliche Stadien und Eier der Grünen Bohnenspinnmilbe (Tetranychus urticae)

Die erfindungsgemäßen Verbindungen werden als wässrige Emulsionen mit der Wirkstoffkonzentration 0,1% eingesetzt. Mit diesen Wirkstoffzubereitungen werden getopfte und künstlich mit Spinnmilben (Tetranychus urticae) besetzte Buschbohnenpflanzen (Phaseolus vulgaris) im Primärblattstadium tropfnaß gespritzt und für 7 Tage im Labor aufgestellt. Danach wird die Mortalität der beweglichen Stadien einerseits und der Eier andererseits mit Hilfe einer vergrößernden Lupe in Prozent geschätzt.

Die Ergebnisse sind in nachstehender Tabelle zusammengestellt:

| Verbindung | Wirkung in % auf bewegliche Stadien/Eier |
|---|---|
| 1-(4-Chlorphenyl)-2,2-difluor-1-[3-(4-fluor-3-phenoxyphenyl)-propyl]-cyclopropan | 100/0 |
| 1-(4-Chlorphenyl)-2,2-difluor-1-[3-(3-phenoxyphenyl)-propyl]-cyclopropan | 86/0 |
| 2,2-Difluor-1-(4-ethoxyphenyl-1-[3-(3-phenoxyphenyl)-propyl]-cyclopropan | 84/0 |
| 2,2-Difluor-1-(4-ethoxyphenyl)-1-[3-(4-fluor-3-phenoxyphenyl)-propyl]-cyclopropan | 100/100 |
| 2,2-Difluor-1-(4-difluormethoxyphenyl)-1-[3-(4-fluor-3-phenoxyphenyl)-propyl]-cyclopropan | 90/96 |
| 2,2-Difluor-1-(4-fluorphenyl)-1-[3-(4-fluor-3-phenoxyphenyl-propyl]-cyclopropan | 100/48 |
| 1-(4-Bromphenyl)-2,2-difluor-1-[3-(4-fluor-3-phenoxyphenyl)-propyl]-cyclopropan | 100/0 |
| 2,2-Difluor-1-[3-(4-fluor-3-phenoxyphenyl)-propyl]-1-(4-trifluormethylphenyl)-cyclopropan | 100/90 |
| 2,2-Difluor-1-[3-(4-fluor-3-phenoxyphenyl)-propyl]-1-[4-(prop-2-yloxyphenyl]-cyclopropan | 100/90 |
| 2,2-Difluor-1-[3-(4-fluor-3-phenoxyphenyl)-propyl]-1-(4-propyloxyphenyl)-cyclopropan | 100/50 |
| 1-(4-Butyloxyphenyl)-2,2-difluor-1-[3-(4-fluor-3-phenoxyphenyl)-propyl]-cyclopropan | 90/0 |
| 2,2-Difluor-1-[3-(4-fluor-3-phenoxyphenyl)-propyl]-1-(4-methylphenyl)-cyclopropan | 82/0 |
| 1-(3,4-Dichlorphenyl-2,2-difluor-1-[3-(4-fluor-3-phenoxyphenyl)-propyl]-cyclopropan | 82/0 |
| 2,2-Difluor-1-(4-ethoxy-3-fluorphenyl)-1-[3-(4-fluor-3-phenoxyphenyl)-propyl]-cyclopropan | 100/100 |
| 2,2-Difluor-1-[4-(2-fluorethoxy)-3-fluorphenyl]-1-[3-(4-fluor-3-phenoxyphenyl)-propyl]-cyclopropan | 100/100 |
| 2,2-Dichlor-1-(4-chlorphenyl)-1-[3-(3-phenoxyphenyl)-propyl]-cyclopropan (DE-OS 33 17 908) | 0/0 |

Anwendungsbeispiel 5

Insektizide bzw. akarizide Wirkung gegen Boophilus microplus (1), Lucilia sericata (2), Musca domestica (3) und Blattella germanica (4)

(1) Filterpapier (9 cm ∅) wird mit 1 ml eines aliquoten Teiles einer Lösung der Testsubstanz in Aceton bei verschiedenen Konzentrationen getränkt. Nach Trocknung werden die Filterpapiere zu Umschlägen gefaltet, in welche Zecken-Larven (Boophilus microplus) eingebracht und für 48 Stunden bei 25°C und 80% Raumfeuchte gehalten werden. Anschließend wird die prozentuale Mortalität der Larven festgestellt und mit den Kontrollversuchen verglichen.
Die Kontrollversuche ergaben eine Mortalität von <5%, währenddessen die Verbindungen gemäß den Beispielen 1 bis 17 eine 90%ige Mortalität bei einer Konzentration von 100 ppm oder weniger ergaben.

(2) 1 ml eines aliquoten Teiles einer Lösung der Testsubstanz in Aceton bei verschiedenen Konzentrationen wird auf Baumwoll-Dentalrollen (1 cm x 2 cm) aufgebracht, die sich in Glasröhrchen (2 cm ∅ und 5 cm Länge) befinden. Nach Trocknung wird das so behandelte Material mit 1 ml einer Nährlösung getränkt, die von Junglarven der Schafsschmeißfliege (Lucilia sericata) durchsetzt ist. Dann werden die Glasröhrchen mit einem Baumwollstopfen verschlossen und für 24 Stunden bei 25°C gehalten.

Die Auswertung der Versuche ergab, daß bei den Kontrollversuchen die Mortalität <5% war, währenddessen die Verbindungen gemäß den Beispielen 1, 3–14 und 16–19 eine $LC_{90}$ bei 100 ppm oder weniger aufwiesen.

(3) Aliquote Teile einer Lösung der Testsubstanz in Aceton in verschiedenen Konzentrationen werden auf Filterpapier (9 cm ∅) in Petrischalen (9 cm ∅) mit Uhrglasabdeckung aufgebracht. Nach Verdampfen des Lösungsmittels werden die so behandelten Oberflächen zusammen mit den Kontrollversuchen, die mit Aceton allein behandelt wurden, ausgewachsenen Hausfliegen (Musca domestica) exponiert und für 24 Stunden bei 22°C gehalten. Die prozentuale Mortalität wird anschließend durch Auszählen bestimmt.

Bei den Kontrollversuchen war die Mortalität <5%, währenddessen die Verbindungen gemäß den Beispielen 1, 3, 4, 6, 9–13 und 16 eine $LD_{90}$ bei 400 mg/$m^2$ oder weniger aufwiesen.

(4) Aliquote Teile einer Lösung der Testsubstanz in Aceton in verschiedenen Konzentrationen werden auf Glasplatten (10 x 10 cm) aufgebracht. Nach Verdampfen des Lösungsmittels werden die so behandelten Oberflächen zusammen mit Kontrollversuchen, die mit Aceton allein behandelt wurden, mit Junglarven (L2) der Deutschen Schabe (Blattella germanica) exponiert, indem sie durch mit Poly-Tetrafluorethylen bedampfte Glasringe (6 cm Ø) auf der behandelten Oberfläche für 24 Stunden bei 22°C gehalten werden. Die prozentuale Mortalität der Insekten wird anschließend durch Auszählen bestimmt.

Bei den Kontrollversuchen war die Mortalität <5%, währenddessen die Verbindungen gemäß den Beispielen 1–18 eine $LD_{90}$ bei 100 mg/$m^2$ oder weniger zeigten.

## Patentansprüche

1. Difluorcyclopropanderivate der allgemeinen Formel I

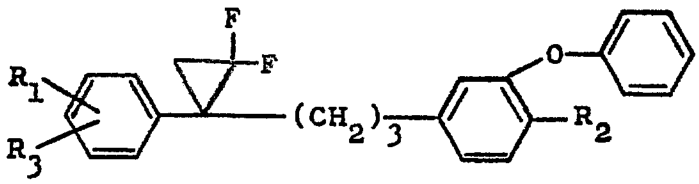

(I),

in der $R_1$ und $R_3$ gleich oder verschieden sind und Wasserstoff, Halogen, $C_{1-4}$-Alkoxy, Fluor-substituiertes $C_{1-4}$-Alkoxy, $C_{1-4}$-Alkyl oder Trifluormethyl und $R_2$ Wasserstoff oder Fluor bedeuten.

2. Difluorcyclopropanderivate gemäß Anspruch 1, worin $R_1$ sich in 4-Stellung befindet und Chlor oder Ethoxy bedeutet und $R_3$ Wasserstoff ist.

3. 1-(4-Chlorphenyl)-2,2-difluor-1-[3-(3-phenoxyphenyl)-propyl]-cyclopropan gemäß Anspruch 2.

4. 2,2-Difluor-1-(4-ethoxyphenyl)-1-[3-(3-phenoxyphenyl)-propyl]-cyclopropan gemäß Anspruch 2.

5. 1-(4-Chlorphenyl)-2,2-difluor-1-[3-(4-fluor-3-phenoxyphenyl)-propyl]-cyclopropan gemäß Anspruch 2.

6. 2,2-Difluor-1-(4-ethoxyphenyl)-1-[3-(4-fluor-3-phenoxyphenyl)-propyl]-cyclopropan gemäß Anspruch 2.

7. Verfahren zur Herstellung von Difluorcyclopropanderivaten gemäß den Ansprüchen 1 bis 6, dadurch gekennzeichnet, daß man eine Verbindung der allgemeinen Formel II

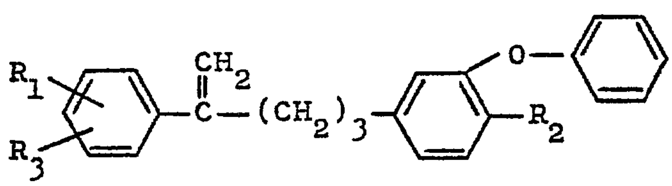

(II)

mit Difluorcarben umsetzt, wobei $R_1$, $R_2$ und $R_3$ die in den Anspüchen 1 bis 6 genannte Bedeutung haben.

8. Schädlingsbekämpfungsmittel, gekennzeichnet durch einen Gehalt an mindestens einer Verbindung gemäß den Ansprüchen 1 bis 6.

9. Mittel zur Bekämpfung von Insekten und Milben gemäß Anspruch 8.

10. Schädlingsbekämpfungsmittel gemäß Anspruch 8 in Mischung mit Träger- und/oder Hilfsstoffen.

## Claims

1. Difluorocyclopropane derivatives of the general formula I

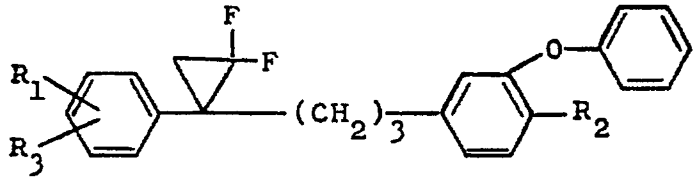

(I),

in which $R_1$ and $R_3$ are the same or different and are hydrogen, halogen, $C_{1-4}$-alkoxy, fluoro-substituted $C_{1-4}$-alkoxy, $C_{1-4}$-alkyl or trifluoromethyl and $R_2$ is hydrogen or fluorine.

2. Difluorocyclopropane derivatives according to claim 1, wherein $R_1$ is in the 4-position and is chlorine or ethoxy and $R_3$ is hydrogen.

3. 1-(4-Chlorophenyl)-2,2-difluoro-1-[3-(3-phenoxyphenyl)-propyl]cyclopropane, according to claim 2.

4. 2,2-Difluoro-1-(4-ethoxyphenyl)-1-[3-(3-phenoxyphenyl)-propyl]cyclopropane, according to claim 2.

5. 1-(4-Chlorophenyl)-2,2-difluoro-1-[3-(4-fluoro-3-phenoxyphenyl)-propyl]cyclopropane, according to claim 2.

6. 2,2-Difluoro-1-(4-ethoxyphenyl)-1-[3-(4-fluoro-3-phenoxyphenyl)-propyl]cyclopropane, according to claim 2.

7. A process for the preparation of difluorocyclopropane derivatives according to claims 1 to 6 characterised by reacting a compound of general formula II

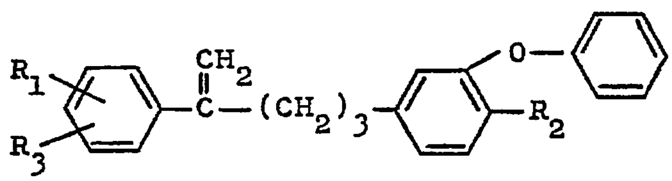

(II)

in which $R_1$, $R_2$ and $R_3$ have the meanings given in claims 1 to 6, with difluorocarbene.

8. Pesticidal compositions characterised in that they comprise at least one compound according to claims 1 to 6.

9. Composition for the control of insects and mites according to claim 8.

10. Pesticidal composition according to claim 8 in admixture with diluent and/or carriers.

**Revendications**

1. Difluorocyclopropanes répondant à la formule générale I:

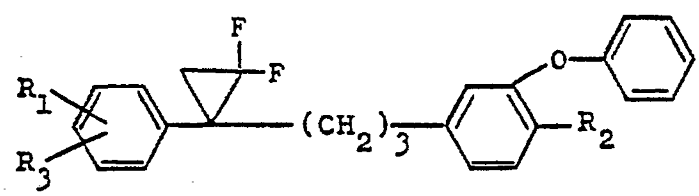

(I)

dans laquelle $R_1$ et $R_3$ représentent chacun, indépendamment l'un de l'autre, l'hydrogène, un halogène, un alcoxy en $C_1$–$C_4$, un alcoxy en $C_1$–$C_4$ fluoré, un alkyle en $C_1$–$C_4$ ou un trifluorométhyle, et $R_2$ représente l'hydrogène ou le fluor.

2. Difluor-cyclopropanes selon la revendication 1 dans lesquels $R_1$ se trouve à la position 4 et représente le chlore ou un radical éthoxy, et $R_3$ représente l'hydrogène.

3. Composé selon la revendication 2, en l'espèce le (chloro-4 phényl)-1 difluoro-2,2 [(phénoxy-3 phényl)-3 propyl]-1 cyclopropane.

4. Composé selon la revendication 2, en l'espèce le difluoro-2,2 (éthoxy-4 phényl)-1 [(phénoxy-3 phényl)-3 propyl]-1 cyclopropane.

5. Composé selon la revendication 2, en l'espèce le (chloro-4 phényl)-1 difluoro-2,2 [(fluoro-4 phénoxy-3 phényl)-3 propyl]-1 cyclopropane.

6. Composé selon la revendication 2, en l'espèce le difluoro-2,2 (éthoxy-4 phényl)-1 [(fluoro-4 phénoxy-3 phényl)-3 propyl]-1 cyclopropane.

7. Procédé pour préparer des difluorocyclopropanes selon l'une quelconque des revendications 1 à 6, procédé caractérisé en ce qu'on fait réagir avec le difluorocarbène un composé répondant à la formule générale II:

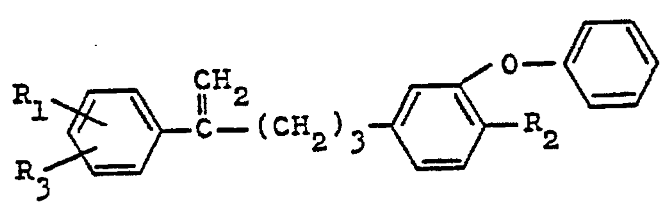

(II)

dans laquelle $R_1$, $R_2$ et $R_3$ ont les significations qui apparaissent dans les revendications 1 à 6.

8. Produits pesticides caractérisés en ce qu'ils contiennent au moins un composé selon l'une quelconque des revendications 1 à 6.

9. Produits pour combattre des insectes ou des acariens, produits qui sont conformes à la revendication 8.

10. Produits pesticides selon la revendication 8 qui contiennent également des supports et/ou des adjuvants.